# EUROPEAN PATENT APPLICATION

(11) **EP 2 524 908 A1**
(43) Date of publication of application: **21.11.2012**
(21) Application number: 11166911.5
(22) Date of filing: 20.05.2011
(51) Int. Cl.: C07C 45/68, C07C 49/67, C07D 295/023

(54) **Process for the preparation of alfa-substituted ketones and their application in synthesis of pharmaceutically active compounds**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to a novel one step preparation of 6-methoxy-2-phenyl-tetralone starting from 6-methoxy-tetralone and its use as an intermediate in the synthesis of pharmaceutically active compounds.

## Description

### FIELD OF INVENTION

The present invention relates to the field of preparative organic chemistry, in particular to a new process for α-arylation of cyclic ketones to provide intermediate compounds for the preparation of nafoxidine, a precursor for lasofoxifene, a non-steroidal selective estrogen receptor modulator (SERM).

### BACKGROUND OF THE INVENTION

Selective estrogen receptor modulators (SERMs) are a group of compounds that are classified to act on estrogen receptors. In contrast to pure receptor agonists and antagonists, SERMs reveal different effects depending on the tissue they are applied to. SERMs thereby offer the possibility to be selectively used in various tissues to inhibit or stimulate estrogen-like actions. Further, some SERMs may act as good replacements for hormone replacement therapy (HRT). One selective estrogen receptor modulator is lasofoxifene which was found to be effective for many applications and can be used against osteoporosis, breast cancer and the vaginal atrophy.

The intermediate compound nafoxidine of the formula **1** and respective salts thereof are key compounds in the synthesis of lasofoxifene of the formula **2** [cis-6-phenyl-5-[4-(2-pyrrolidine-1-ylethoxy}phenyl]-5,6,7,8-tetrahydronaphthalene-2-ol]. In WO 96121fi56 A1 nafoxidine as hydrochloride adduct is converted by hydrogenation to cis-1-{2-[4-{6-methoxy-2-phenyl-1,2,3,4-tetrahydronaphthalene-1-yl}phenaxy]ethyl}pyrrolidine which is subsequently demethylated with borontribromide to yield lasofoxifene. In WO 96/21656 there is also disclosed a preparation of the key intermediate nafoxidine hydrochloride which can be prepared in three steps starting from 6-methoxy-1-tetralone. Said starting material is converted with 1-[2-(4-bromophenoxy)ethyl]pyrrolidine to the intermediate compound [1-{2-[4-(6-methoxy-3,4-dihydronaphthalene-1-yl)phenoxy]ethyl}pyrrolidine] which is subsequently monobrominated and then further converted to nafoxidine hydrochloride - see Scheme 1.

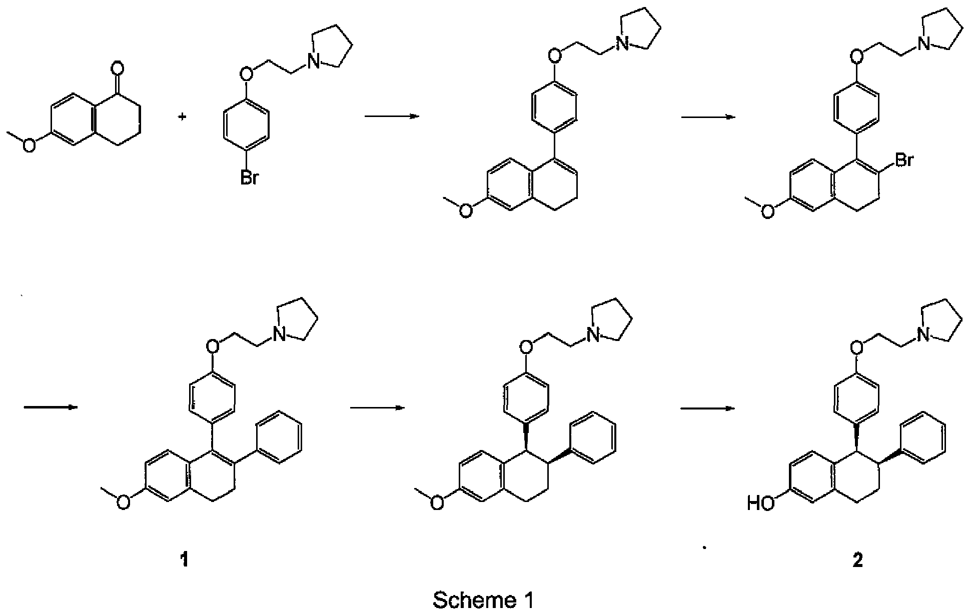

This synthetic route for nafoxidine hydrochloride is already a multistep protocol which comprises three reaction steps which all require isolation and purification of the corresponding products. Therefore simplification and shortening of the synthesis of nafoxidine and related pharmaceuticallly active compounds are an object of the present invention, particularly in view of an economically reasonable preparation of lasofoxifene.

### SUMMARY OF THE INVENTION

The object is solved by the present invention owing to the provision of a process for an preparation of the key intermediate compound 6-methoxy-2-phenyl-3,4-dihydronaphthalene-1(2H)-one of the formula (III) by arylation of 6-methoxy-3,4-dihydronaphthalene-1(2H)-one (I) with phenyl halides (II).

Proceeding via said acylation simplifies and shortens the process for the preparation of nafoxidine to a two step process starting from a simple and cheap material 6-methoxy-3,4-dihydronaphthalene-1 (2H)-one of the formula (I). By following the synthetic route of the present invention the preparation of nafoxidine in WO 96/21656 A1 can be reduced by one step. A preparative route for nafoxidine as well as for lasofoxifene being simplified and shortened by at least one step is especially industrially useful since also the number of isolation and purification steps can be reduced simultaneously.

It is particularly surprising that upon all theoretically possible synthetic routes to prepare either nafoxidine or lasofoxifene via numerous potential intermediates a useful intermediate could be found that not only shortens and simplifies the preparation of said compounds, but also can be prepared in high yield.

Aspects, advantageous features and preferred embodiments of the present invention summarized in the following claims, respectively alone or in combination, contribute to solving this and other objects of the invention:
1. A process for the preparation of a compound of the formula (III) 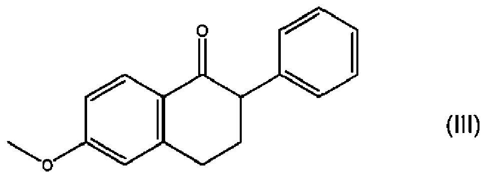 comprising
   reacting a cyclic ketone of the formula (1) 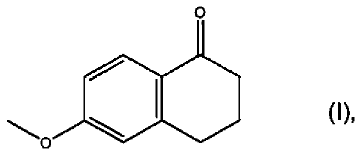 with an aryl halide compound of the formula (II) 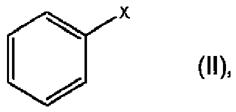 wherein X is selected from chlorine, bromine and iodine,
   to yield the α-arylated ketone of the formula (III).
   It was surprisingly found that the intermediate compound 6-methoxy-2-phenyl-3,4-dihydronaphthalene-1(2H)-one of the formula (III) can not only be readily prepared from 6-methoxy-3,4-dihydronaphthalene-1(2H)-one by catalytic arylation, but also in high, almost quantitative yield. Preferably the yield of compound (III) in the process of item 1 is at least 85%, more preferably the yield is at least 90 % with respect to the starting material 6-methoxy-3,4-dihydronaphthalene-1 (2H)-one.
2. The process according to item 1, wherein the reaction is performed in the presence of a metal catalyst.
3. The process according to item 1 or 2, wherein the reaction is performed in the presence of a catalyst of the generic formula M(Y)ₚ(L)_{q}, wherein M is a transition metal, Y is an inorganic anion, L is a coordinating ligand, p is an integer from 0 to 4 and q is an integer from 0 to 4 with the proviso that q and p cannot be 0 at the same time.
4. The process according to item 2 or 3, wherein the metal or transition metal M is selected from the group consisting of palladium, platinum, nickel, iridium, rhodium and copper, preferably palladium.
5. The process according to item 3, wherein the anion Y is selected from the group consisting of fluoride, chloride, bromide, iodide, acetate, nitrate, sulfate, sulfide, tosylate and triflate, preferably chloride and bromide.
6. The process according to item 3, wherein the ligand L is selected from the group consisting of phosphines, amines, imines.
7. The process according to item 3, wherein the ligand L is a chelating phosphine.
8. The process according to item 3, wherein the ligand L is selected from the group consisting of triphenyl phosphine, tri-tert.-butyl phosphine and di-tert.-butyl-(4-dimethylamino phenyl)phosphine.
9. The process according to item 3, wherein the ligand L is di-*tert*.-butyl-(4-dimethylamino phenyl)phosphine.
10. The process according to any one of the preceding items, wherein the reaction is performed in the presence of a catalyst of the formula (IV) 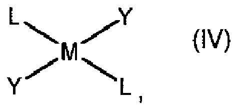 wherein M is palladium, L is di-*tert*.-butyl-(4-dimethylamino phenyl)phosphine and Y is chloride or bromide.
11. The process according to any item 2 to 10, wherein the amount of catalyst used ranges from 0.01 mol%to 2 mol% with respect to the starting material of the formula (I).
12. The process according to any item 2 to 11, wherein the amount of catalyst used ranges from 0.05 mol%to 1.5 mol% with respect to the starting material of the formula (I), preferably from 0.1 mol% to 1 mol%.
13. The process according to any of the preceding items, wherein the reaction is carried out in the presence of a base.
14. The process according to item 13, wherein the base used for the arylation is selected from the group consisting of inorganic basic salts comprising alkoxides, carbonates, hydrides, hexaalkyldisilazanes and hydroxides.
15. The process according to item 13 or 14, wherein the base used for the arylation is selected from the group consisting of alkoxides comprising alkaline, alkaline earth and earth metal alkoxides, preferably potassium alkoxides and sodium alkoxides, most preferably sodium alkoxides.
16. The process according to any item 13 to 15, wherein the base used for the arylation is selected from the group consisting of alkoxides comprising methoxides, ethoxides, isopropoxides, butoxides and tert-butoxide.
17. The process according to any item 13 to 16, wherein the base used for the arylation is sodium *tert*-butoxide.
18. The process according to item 13, wherein the base used for the arylation is selected from the group consisting of organic bases comprising amides, amidines, tertiary amines including pyridine and triethylamine.
19. The process according to any one of the proceeding items 13 to 18, wherein the base is used in excess with respect to the starting material of the formula (I), preferably in 1.5 to 5 fold excess.
20. The process according to any one of the proceeding items, wherein the reaction is performed in an organic solvent or mixtures comprising organic solvents.
21. The process according to item 20, wherein the reaction is performed in dioxane, toluene, tetrahydrofurane, 2-methyl tetrahydrofurane, dimethoxyethane, methyl tert-butyl-ether, preferably in toluene or dioxane.
22. The process according to any one of the proceeding items, wherein the reaction is performed at ambient temperatures to elevated temperatures, preferably at elevated temperatures.
23. The process according to item 22, wherein the reaction is performed at elevated temperatures above ambient temperatures and below the boiling point of the solvent or solvent mixture used, or is performed under reflux conditions.
24. The process according to item 22 or 23, wherein the reaction is performed between 55 and 65°C in toluene, preferably at 60 °C.
25. The process according to item 22 or 23, wherein the reaction is performed between 95 °C to the boiling temperature in dioxane, preferably at 100 °C.
26. The process according to any one of the preceding items, wherein the starting material of the formula (I) and the aryl halide compound of the formula (II) are used in a substantially equimolar ratio.
27. The process according to any one of the preceding items, wherein in the aryl halide compound of the formula (II), X is chlorine or bromine.
28. The process according to any one of the preceding items, wherein the product of the formula (III) is separated from the reaction mixture.
29. The process according to any one of the preceding items, wherein the reaction is performed under substantially air free conditions or under substantially oxygen free conditions.
30. The process according to any one of the preceding items, wherein the reaction is performed in an atmosphere substantially consisting of nitrogen.
31. The process according to any one of the preceding items, wherein the compound of the formula (III) is prepared in a yield of at least 75% yield, preferably at least 85% yield, more preferably at least 90% yield.
32. The process according to any one of the preceding items further comprising subjecting the arylated product of the formula (III) to further reaction steps to prepare a pharmaceutical active compound.
33. The process according to any one preceding items, wherein the compound of the formula (III) is converted by reaction with the aryl halide of the formula (V) or a salt thereof 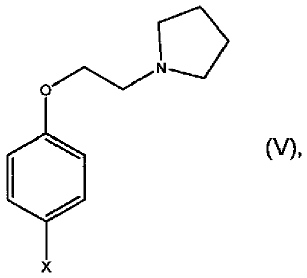 wherein X is selected from chlorine, bromine and iodine, to yield a compound of the formula (VI) or a salt thereof, 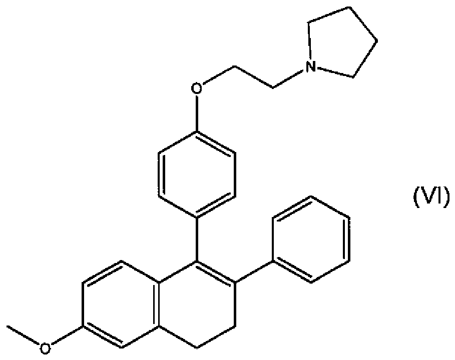 optionally converting the compound of the formula (VI) to a pharmaceutically acceptable salt.
   The suitability of the compound of the formula (III) as an intermediate in the synthesis of a pharmaceutically active compound such as nafoxidine or lasofoxifene is demonstrated by the conversion of (III) with the aryl halide compound of the formula (V). In a preferred embodiment the aforementioned conversion is performed by treating compound (V) with a strong base in the presence of a rare earth halide. The resulting derivative of (V) is then further reacted with compound (III) to yield a compound of the formula (VI). Thereby, a simple and short two step synthesis of nafoxidine starting from 6-methoxy-tetralone is effected according to an aspect of the present invention.
34. Use of the compound of the formula {III) as intermediate for the preparation of nafoxidine or a salt thereof. 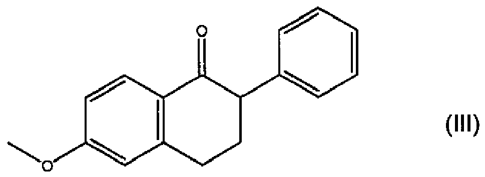
35. Use of the compound of the formula (III) as intermediate for the preparation of lasofoxifene or a salt thereof. 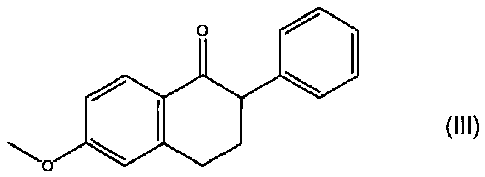
36. A process for the preparation of a compound of the formula (VII) or a salt thereof, comprising :
   (i) converting a compound of the formula (I) to the arylated compound of the formula (III) or a salt thereof by a process according to any one of item 1 to 33; 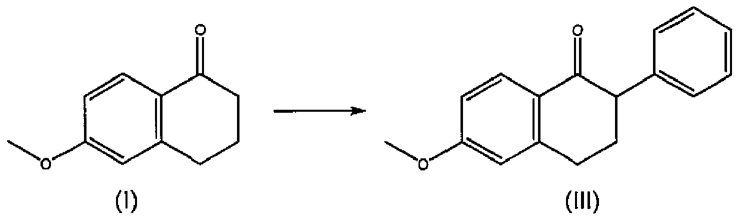
   (ii) converting the compound of the formula (III) or a salt thereof by reaction with a compound of the formula (V) to prepare a compound of the formula (VI) or a salt thereof; 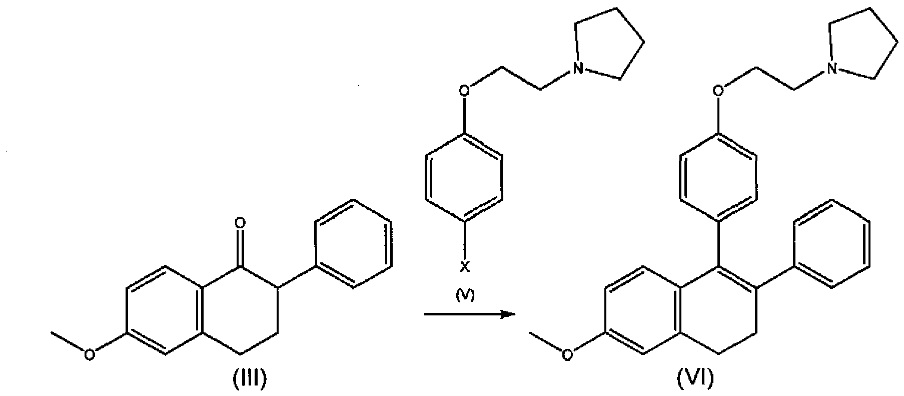 wherein X is selected from, chlorine, bromine and iodine, preferably bromine (iii) further converting the compound of the formula (VI) or a salt thereof to a compound of the formula (VII) or a salt thereof. 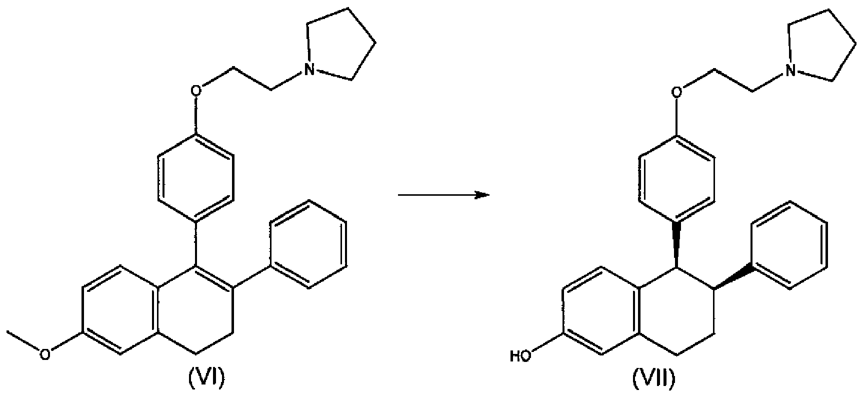
37. A process for the preparation of a pharmaceutical composition comprising nafoxidine or a salt thereof as active ingredient, comprising the steps of:
   a) preparing nafoxidine or a salt thereof according to the process according to item 33, and
   b) admixing the thus prepared nafoxidine or a salt thereof with at least one pharmaceutically acceptable excipient.
38. A process for the preparation of a pharmaceutical composition comprising lasofoxifene or a salt thereof as active ingredient, comprising the steps of:
   a) preparing lasofoxifene or a salt thereof according to the process according to item 36, and
   b) admixing the thus prepared iasofoxifene or a salt thereof with at least one pharmaceutically acceptable excipient.

### DETAILED DESCRIPTION

The present invention is now described in more detail by referring to further preferred and further advantageous embodiments and examples, which are however presented for illustrative purposes only and shall not be understood as limiting the scope of the present invention.

In order to find a more efficient and shorter way to prepare nafoxidine or a salt thereof it was surprisingly found that the intermediate compound 6-methoxy-2-phenyl-3,4-dihydronaphthalene-1(2H)-one of the formula (III) can not only be readily prepared from 6-methoxy-3,4-dihydronaphthalene-1(2H)-one by catalytic arylation, but also in high, almost quantitative yield. Said intermediate can subsequently be conveniently converted to nafoxidine or a salt thereof, which in turn may represent a key intermediate and can be used by itself or for the subsequent synthesis of lasofoxifene.

Syntheses of the compound of formula (III) have been previously disclosed, however using low yield processes and directed to purposes other than preparing nafoxidine or lasofoxifene. In J. Med. Chem. 1988, 31, 107 the compound of formula (III) is prepared by a two step process starting from a 2,4-diphenylbutyroniltrile in 45% yield. In J. Chem. Soc., Perkin Trans. 1, 1997, 1511, 6-methoxy-1-tetralone is first brominated to yield 2-bromo-6-methoxy-1-tetralone which is subsequently converted with a phenyl Grignard reagent to the compound of the formula (III) in 13% yield. In J. Chem. Soc., Perkin Trans. 1, 2000, 3775, 6-methoxy-1-tetralone is prepared as mixture with 2,2-diphenyl-6-methoxy-1-tetralone and starting material by reaction of 6-methoxy-1-tetralone with an exotic biphenyl-2,2'-ylenebismuth phenyl complex in 18% yield. Since all previously disclosed syntheses exhibit only yields lower than 50% and partly require extensive isolation and purification none of these processes appear useful for an industrial application.

Catalytic arylations of cyclic benzoketones using transition metal complexes are known in the art per se (see e.g. Chem. Rev., 2010, 110, 1147; Angew. Chem. lnt. Ed., 2010, 49, 676; J. Am. Chem. Soc., 2001, 123, 5816; Org. Proc. Res. Dev., 2008, 12, 522; J. Org. Chem., 2006, 71, 685; Org. Lett., 2007, 9, 5489; Org Lett., 2010, 12, 1032), however none of these reactions were performed with respect to the preparation of nafoxidine or lasofoxifene or appropriate intermediates thereof.

Compared with the conventional low yield syntheses of the compound of the formula (III), the present invention surprisingly satisfies a higher to unmet need for an improvement of processes for the preparation of a compound that is suitable for industrial production of nafoxidine and lasofoxifene.

According to one aspect of the present invention, the compound of the formula (III) can be prepared with very high efficiency by reacting a cyclic ketone of the formula (I) with an aryl halide compound of the formula (II), wherein X is selected from chlorine, bromine and iodine, preferably is chlorine or bromine. Thereby, the process of the present invention provides the preparation of the compound of the formula (III) from the ketone of the formula (I) in one single reaction step without the use of uncommon phenyl nucleophiles as disclosed in J. Chem. Soc., Perkin Trans. 1, 2000, 3775.

It was surprisingly found that the compound of the formula (III) can be readily prepared in high, almost quantitative yield. In this regard, the yield of the compound of the formula (III) is preferably at least 75%, more preferably at least 85%, most preferably at least 90% with respect to the starting material of the formula (I). The process of the present invention is therefore a simple and short, high yield process for the preparation of the compound of the formula (III) with the compound of the formula (I) as starting material. Also, the process of the present invention is industrially very useful.

In a preferred embodiment of the present invention, the process for the preparation of the compound of the formula (III) from the compound of the formula (I) is performed in the presence of a metal catalyst. In a further preferred embodiment, the process of the present invention is performed in the presence of a transition metal catalyst, notably of the generic formula M(Y)p(L)q, wherein M is a transition metal, Y is an inorganic anion, L is a coordinating ligand, p is an integer from 0 to 4 and q is an integer from 0 to 4 with the proviso that q and p can not be 0 at the same time. Suitable metals/transition metals are palladium, platinum, nickel, iridium, rhodium or copper. The anion Y can be selected from the group consisting of fluoride, chloride, bromide, iodide, acetate, nitrate, sulphate, sulphide, tosylate and triflate. The ligand L can be selected from a ligand that is suitable to coordinate to a transition metal atom. Thereby, the ligand can be monodentate or polydentate. Preferably, the ligand L is selected from the group consisting of phosphines, amines and imines. In case the ligand L is a polydentate ligand, the ligand can act as a chelating ligand. For example, the ligand can be a chelating phosphine. In a more preferred embodiment, the ligand L is selected from a group of bulky, sterically demanding phosphines, for example triphenyl phosphine, tri-*tert*.-butyl phosphine and di-*tert*.-butyl-(4-dimethylamino phenyl)phosphine.

Further, the catalyst of the generic formula M(Y)ₚ(L)_{q} is in terms of the transition metal M not restricted to a specific coordination and can adopt a chemically reasonable geometry. In a preferred embodiment, the catalyst of the generic formula M(Y)ₚ(L)_{q} adopts a tetrahedral or square planar geometry, more preferably adopts a square planar geometry.

In further preferred embodiment of the present invention, the catalyst has the formula (IV) 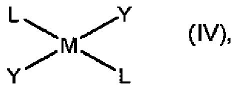 wherein M is palladium, L is di-*tert*.-butyl-(4-dimethylamino phenyl)phosphine and Y is chloride or bromide.

In view of an economically reasonable and industrially applicable process, the process according to the present invention allows the potentially expensive metal catalyst to be used in a very small ratio in comparison to the starting material of a formula (I). In a preferred embodiment of the present invention the catalyst used is applied in an amount from 0.01 to 2 mol% with respect to the starting material of formula (I). In a more preferred embodiment, the amount of the catalyst used ranges from 0.05 to 1.b mol% with respect to the starting material of the formula (I), in particular from 0.1 mol% to 1 mol%.

In a further embodiment of the present invention, the reaction to prepare the compound of the formula (III) according to the present invention is performed in the presence of a base. This base can be selected from the group consisting of inorganic basic salts comprising alkoxides, carbonates, hydrides, hexaalkyldisilazanes and hydroxides. Inorganic basic salts bear the benefit of being cheap and practical when used in large scale.

In a further preferred embodiment the process of the present invention is performed in the presence of an alkoxide selected from the group consisting of alkaline, alkaline earth and earth metal alkoxides. In a more preferred embodiment, the base is selected from potassium alkoxides and sodium alkoxides, most preferably sodium alkoxides.

In a further preferred embodiment the alkoxides originate from simple alcohols such as methanol, ethanol, isopropanol, butanol and ted-butanol. Hence, the preferred alkoxides used for the process of the present invention are methoxides, ethoxides, isopropoxides, butoxides and *tert* butoxides. In a most preferred embodiment, the base used for the arylation of the present invention is sodium *tert*-butoxides. Further, the base for the arylation of the present invention can also be selected from the group consisting of organic bases selected from amides, amidines, tertiary amines including pyridine and triethylamine.

Preferably, the base for the process of the present invention is used in excess with respect to the starting material of the formula (I). The excess of the base can vary according to the applied conditions. Preferably, the base is used in 1.5 to 5 fold excess.

The preparation of the compound of the formula (III) according to the present invention is performed in an organic solvent or mixtures comprising organic solvents.

Suitable organic solvents are for example ethers, aromatic hydrocarbons, aliphatic hydrocarbons and halogenated hydrocarbons. The organic solvent or solvents is (are) preferably selected from the group consisting of diethyl ether, methyl *tert*-butyl ether, tetrahydrofuran, dioxane, C5-C12 linear hydrocarbons, cyclohexane, decalin, benzene, toluene, dichloromethane, chloroform. More preferably, the preparation of the compound of the formula (III) according to the present invention is performed in toluene or in dioxane.

Further, the reaction is suitably performed at ambient to elevated temperatures, preferably at elevated temperatures. Accordingly, the reaction temperature can range from ambient temperatures to elevated temperatures, which are below the boiling point of the solvent or the solvent mixture used. Another suitable option of performing the reaction is to apply reflux conditions. In a preferred embodiment of the present invention the preparation of the compound of the formula (III) is performed between 55 and 65 °C in toluene, preferably at 60°C. Another preferred embodiment of said reaction is to apply temperatures between 95 °C to the boiling temperature in dioxane, preferably at a 100°C.

In a further preferred embodiment of the present invention the starting material of the formula (I) and the aryl halide compound of the formula (II) are used in substantially equimolar ratio. The term "substantially equimolar ratio" is to be understood that it is the intention to run the reaction of the two starting materials in equimolar ratio. However, imprecise weighing of starting materials, improperly working balances, humidity, moisture in the starting materials, any kind of impurities in the starting materials and related factors can lead to deviations from an "equimolar ratio". Therefore, the term "substantially equimolar ratio" comprises ratios wherein the amount of one component deviates not more than 10% from the amount of the other component.

The aryl halide compound of the formula (II) is to be understood as a halogen-substituted benzene derivative. Therefore, the substituent X in the formula (II) is a halogen selected from the group consisting of chlorine, bromine and iodine. Preferably, X is chlorine or bromine.

In a further preferred embodiment of the present invention the preparation of the compound of the formula (III) is performed in atmosphere substantially being air free and oxygen free as well as substantially consisting of an inert gas (for example nitrogen or argon). The terms "substantially air free", "substantially oxygen free" and "substantially consisting of nitrogen", all refer atmospheres in reaction vessels which are intended to consist of an inert gas by exchange of atmospheres by conventionally applied techniques. Common procedures to exchange atmospheres in reaction vessels comprise purging the reaction vessel for a certain period of time with the desired inert gas or apply various cycles of evacuating and refilling the reaction vessel with the desired inert gas. It is commonly accepted that upon application of such techniques reaction vessels can be provided with an atmosphere that does not react with sensitive compounds. However, it is further known, that in such atmospheres residual amounts of nitrogen, oxygen or air can remain, even though it has been diligently taken care to prevent this.

In a further aspect of the present invention the compound of the formula (III) can be used as an intermediate for the preparation of pharmaceutically active compounds. Beneficially, the compound of the formula (III) can be used in a one-step reaction to prepare nafoxidine of the formula (VI) by conversion with the aryl halide of the formula (V). The conversion of the compound of the formula (III) to nafoxidine of the formula (VI) is preferably carried out in the presence of a derivative of the compound of the formula (V), which was previously prepared by reacting the phenyl halide of the formula (V) with a strong base and the presence of a rare earth metal halide. In this preferred embodiment nafoxidine of the formula (VI) can thereby be isolated or separated directly from the reaction mixture, and optionally further purified facilitating a one-step synthesis from the starting material of the formula (III). Such isolated and optionally purified nafoxidine of the formula (VI) can then optionally be converted to a pharmaceutically acceptable salt. Therefore, a particular aspect of the present invention is the provision of a short and simple two-step synthesis of nafoxidine of the formula (VI) starting from the cyclic ketone of the formula (I) and proceeding via the intermediate of the formula (III).

In further aspect of the present invention, nafoxidine of the formula (VI) prepared as desired above can further be converted to a compound of the formula (VII) or a salt thereof. Such a conversion from nafoxidine of the formula (VI) to a compound of the formula (VII) (lasofoxifene) can be performed according to known literature procedures in the art, such as for example disclosed in WO 96/21656 A1 incorporated herein by reference. In a further aspect of the present invention, the compound of the formula (III) can therefore be used as an intermediate for the preparation of lasofoxifene or a salt thereof.

A particularly preferred embodiment of the present invention is the preparation of a compound of the formula (VII) or a salt thereof, comprising:
(i) converting a compound of the formula (I) to the arylated compound of the formula (III) or a salt thereof by a process according to the present invention; 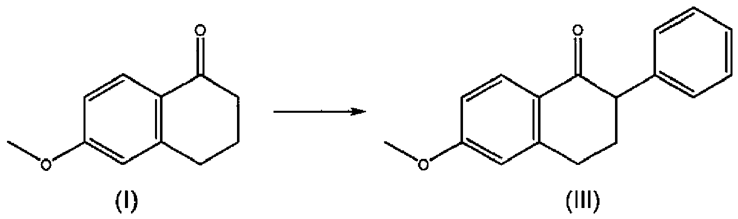
(ii) converting the compound of the formula (III) or a salt thereof by reaction with a compound of the formula (V) according to the present invention to prepare a compound of the formula (VI) or a salt thereof; 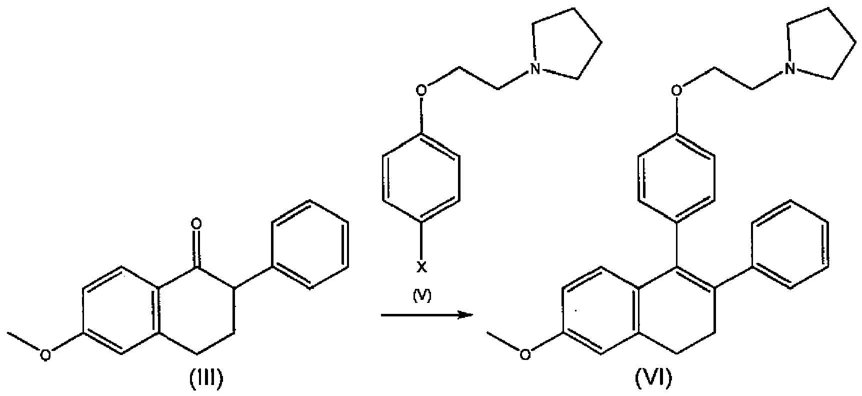 wherein X is selected from fluorine, chlorine, bromine and iodine,
(iii) further converting the compound of the formula (VI) or a salt thereof to lasofoxifene of the formula (VII) or a salt thereof.

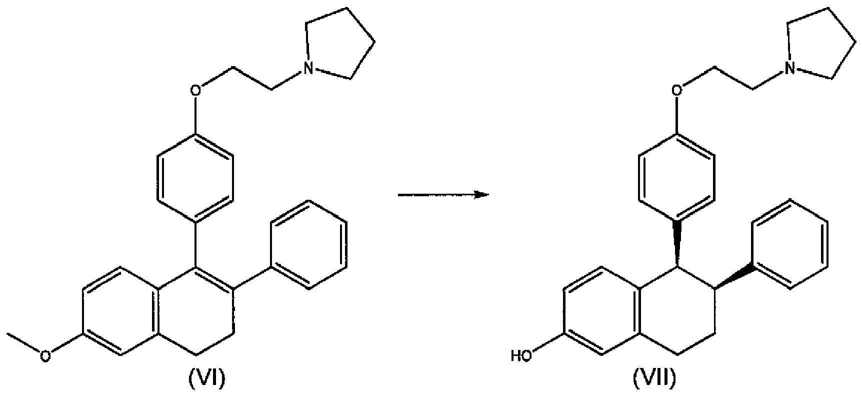

A further aspect of the present invention is the provision of a process for the preparation of a pharmaceutical composition either comprising nafoxidine or lasofoxifene or the respective salts thereof as active ingredient.

For preparing a pharmaceutical composition comprising nafoxidine or lasofoxifene or the respective pharmaceutically acceptable salts thereof as active ingredient, first nafoxidine or lasofoxifene or the respective pharmaceutically acceptable salts thereof is provided by the process as described above proceeding via the intermediate of the formula (III).

Then, the thus prepared nafoxidine or lasofoxifene or the respective pharmaceutically acceptable salts thereof is suitably admixed with at least one suitable pharmaceutically acceptable excipient. Pharmaceutically acceptable excipients may be selected from the group consisting of binders, diluents, disintegrating agents, stabilizing agents, preservatives, lubricants, fragrances, flavoring agents, sweeteners and other excipients known in the field of the pharmaceutical technology.

For example, suitable excipients may be selected from the group consisting of lactose, microcrystalline cellulose, cellulose derivatives, such as hydroxypropylcellulose, polyacrylates, calcium carbonate, starch, colloidal silicone dioxide, sodium starch glycolate, talc, magnesium stearate, polyvinylpyrrolidone, polyethylene glycol and other excipients known in the field of the pharmaceutical technology.

### EXAMPLES

The terminology "Pd-132" used in the following examples refers to a palladium dichloride catalyst of the formula as follows:

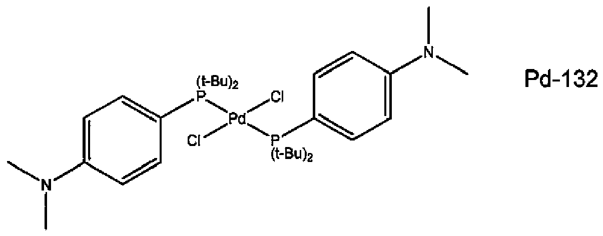

### 1. Comparative example: Evaluation of Pd catalysts

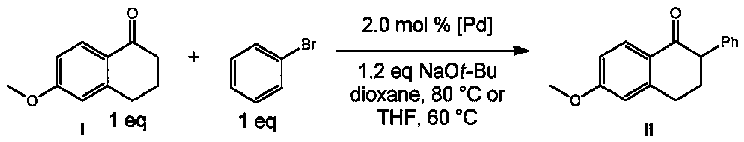

6-Methoxy-3,4-dihydronaphthalene-1(2H)-one (1, 2.0 mmol), NaOt-Bu (2.2 mmol) and [Pd] (0.04 mmol) were added to a Schlenk flask. The flask was sealed with a rubber stopper, evacuated and backfilled with nitrogen three times. Bromobenzene (2.0 mmol) followed by dioxane (2.0 mL, entries 1,2,7) or THF (2.0 mL, entries 3, 4, 5, 6), was added via airtight syringe through the rubber stopper, and the reaction mixture was stirred at 80°C (dioxane) or at 60 °C (THF) for 18 hours. The conversion was monitored by HPLC. The obtained results are shown in Table 9.

**Table 1. Evaluation of Pd catalysts**

| Entry | Pd catalyst | Ketone I | Ketone III |
|---|---|---|---|
| 1 | Pd-113 | 25.35 | 44.20 |
| 2 | Pd-116 | 22.82 | 40.87 |
| 3 | Pd-106 | 59.81 | 14.16 |
| 4 | Pd-100 | 78.51 | 4.55 |
| 5 | Pd-132 | 4.40 | 80.40 |
| 6 | Pd(AmPhos)₂ | 69.93 | 22.66 |
| 7 | Pd-118 | 17.52 | 76.22 |

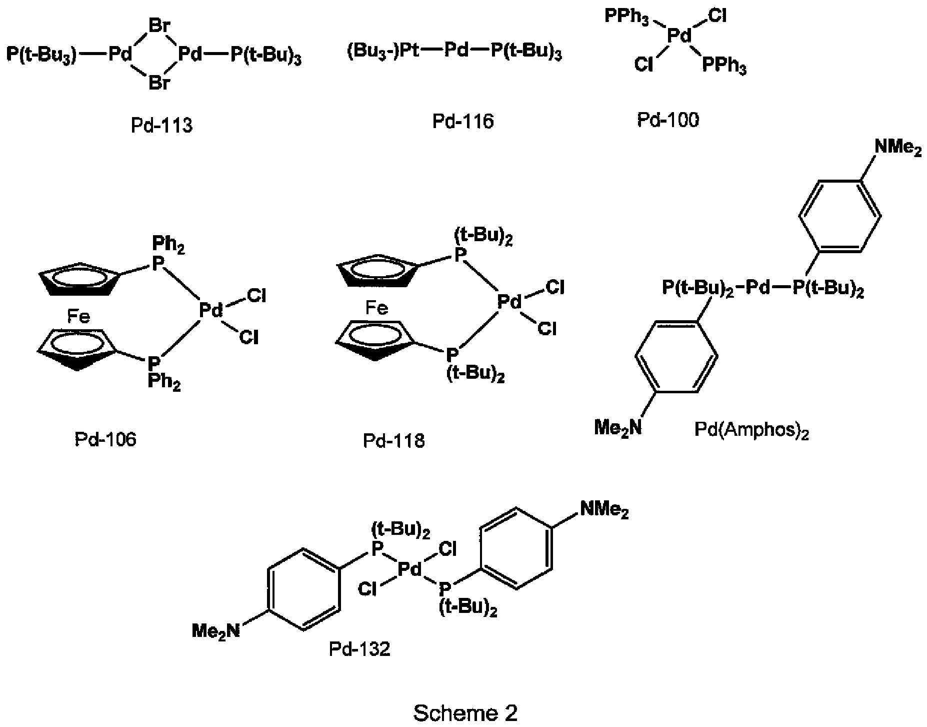

### 2. Synthesis of 6-methoxy-2-phenvl-3,4-dihvdronaphthalene-1(2H)-one (II)

A) 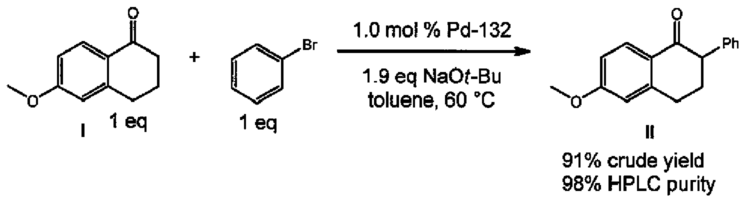 6-Methoxy-3,4-dihydronaphthalene-1(2H)-one (1, 352 mg, 2.0 mmol), NaOt-Bu (365 mg, 3.8 mmol) and Pd-132 (14 mg, 0.02 mmol) were added to a Schlenk flask. The flask was sealed with a rubber stopper, evacuated and backfilled with nitrogen three times. Bromobenzene (0.21 mL, 2.0 mmol) followed by toluene (2.0 mL), was added via airtight syringe through the rubber stopper, and the reaction mixture was stirred at 60°C for 18 hours. The reaction mixture was filtered on silica gel and the silica pad was washed with MTBE (20 mL). The filtrate was concentrated to give the desired product as an off-white solid (461 mg, 91 %).
   ¹H NMR (CDCl₃) δ = 2.39 - 2.44 (m, 2H), 3.0 - 3.1 (m, 2H), 3.8 (t, 1 H), 3.9 (s, 3H), 6.7 (d, 1 H), 6.9 (dd, 1H), 7.2 (m, 2H), 7.3 (m, 1 H), 7.4 (m, 2H), 8.1 (d, 1 H) ppm.
   ¹³C NMR (CDCl₃) δ = 28.9, 31.2, 54.0, 55.4, 112.4, 113.2, 126.4, 126.7, 128.35, 128.4, 130.2, 140.0, 146.5, 163.5, 196.9 ppm.
B) 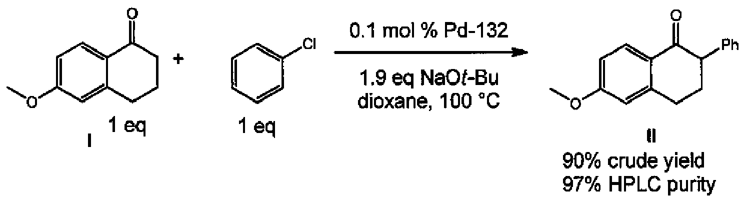 6-Methoxy-3,4-dihydronaphthalene-1(2H)-one (I,352 mg, 2.0 mmol), NaOt-Bu (365 mg, 3.8 mmol) and Pd-132 (1.4 mg, 0.002 mmol) were added to a Schlenk flask. The flask was sealed with a rubber stopper, evacuated and backfilled with nitrogen three times. Chlorobenzene (0.20 mL, 2.0 mmol) followed by dioxane (2.0 mL), was added via airtight syringe through the rubber stopper, and the reaction mixture was stirred at 100°C for 18 hours. The reaction mixture was filtered on silica gel and the silica pad was washed with MTBE (20 mL). The filtrate was concentrated to give the desired product as an off-white solid (455 mg, 90%).
   ¹H NMR (CDCl₃) δ = 2.39 - 2.44 (m, 2H), 3.0 - 3.1 (m, 2H), 3.8 (t, 1 H), 3.9 (s, 3H), 6.7 (d, 1 H), 6.9 (dd, 1 H), 7.2 (m, 2H), 7.3 (m, 1 H), 7.4 (m, 2H), 8.1 (d, 1 H) ppm.
   ¹³C NMR (CDCl₃) δ = 28.9, 31.2, 54.0, 55.4, 112.4, 113.2, 126.4, 126.7, 128.35, 128.4, 130.2, 140.0, 146.5, 163.5, 196.9 ppm.

### 3. Synthesis of 1-(2-(4-(6-methoxy-2-phenyl-3,4-dihydronaphthalen-1-yl)phenoxy)ethynpyrrolidine (III, nafoxidine)

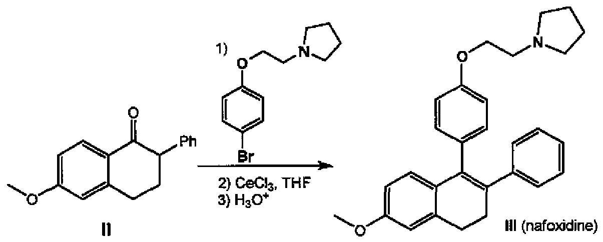

A mixture of anhydrous CeCl₃ (13.6 g, 55.0 mmol) and THF (50 mL) was vigorously stirred for 2h. In a separate flask, a solution of 1-[2-(4-bromophenoxy)ethyl)pyrrolidine (7.7 mL, 36.0 mmol) in THF (100 mL) was cooled to -78 °C and n-BuLi (2.5 M in hexane, 16.9 mL, 44.0 mmol) was slowly added over 10 min. After 15 min. the solution was added to the CeCl₃ slurry cooled at -78°C via cannula and the reaction was stirred for 2 h at -78°C. A solution of 6-methoxy-2-phenyl-3,4-dihydronaphthalene-1(2H)-one (II, 9.2 g, 36.0 mmol) in THF (100 mL) at room temperature was added to the arylcerium reagent via cannula. The reaction was allowed to warm slowly to room temperature and was stirred for 16 h. The mixture was filtered through a celite pad. The filtrate was concentrated *in vacuo* and 3M HCl (40 mL) and Et₂O (200 mL) were added. After stirring for 15 min. the layers were separated. The aqueous layer was further washed with Et₂O (60 mL) and MeTHF (200 mL). The combined organic layers were dried (MgSO₄), filtered and concentrated to provide 6-methoxy-2-phenyl-3,4-dihydronaphthalene-1(2H)-one (II, 4.94 g, 54 %). The aqueous layer was basified to pH 12 with 5 M NaOH. The aqueous mixture was extracted with CH₂Cl₂ (2 x 200 mL). The organic solution was dried (MgSO₄), filtered and concentrated to provide brown oil (7.16 g). Impurities were distilled off by Kugelrohr (100-150 °C, 0.3 mbar) to yield the product (4.33 g; 28 %).
¹H NMR (CDCl₃) δ = 1.8 (m, 4H), 2.6 (m, 4H), 2.8 (m, 2H), 2.9 (m, 4H), 3.8 (s, 3H), 4.1 (t, 2H), 6.6 (dd, 1 H), 6.7 (m, 4H), 7.1 (m, 7H) ppm.
¹³C NMR (CDCl₃) δ = 23.4, 28.9, 30.7, 54.7, 55.1, 55.2, 66.8, 110.7, 113.1, 114.0, 125.5, 127.4, 127.5, 128.2, 130.4, 132.02, 132.04, 134.1, 134.7, 137.6, 143.2, 157.3, 158.3 ppm.

## Claims

1. A process for the preparation of a compound of the formula (III) 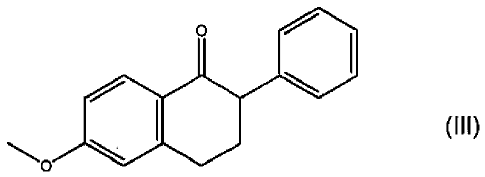 comprising
reacting a cyclic ketone of the formula (I) 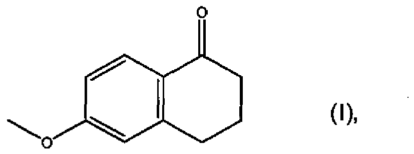 with an aryl halide compound of the formula (II) 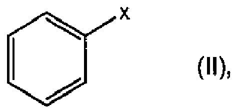 wherein X is selected from chlorine, bromine and iodine,
to yield the α-arylated ketone of the formula (III).

2. The process according to claim 1, wherein the reaction is performed in the presence of a metal catalyst, preferably of a catalyst of the generic formula M(Y)ₚ(L)_{q}, wherein M is a transition metal, Y is an inorganic anion, L is a coordinating ligand, p is an integer from 0 to 4 and q is an integer from 0 to 4 with the proviso that q and p cannot be 0 at the same time.

3. The process according to claim 1 or 2, wherein the reaction is performed in the presence of a catalyst of the formula (IV) 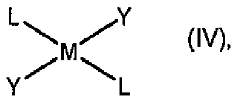 wherein M is palladium, L is di-tert.-butyl-(4-dimethylamino phenyl)phosphine and Y is chloride or bromide.

4. The process according to claim 2 or 3, wherein the amount of catalyst used ranges from 0.05 to 1.5 mol% with respect to the starting material of the formula (I), preferably from 0.1 mol% to 1 mol%.

5. The process according to any of the preceding claims, wherein the reaction is carried out in the presence of a base, preferably selected from the group consisting of alkoxides comprising alkaline, alkaline earth and earth metal alkoxides, more preferably selected from potassium alkoxides and sodium alkoxides.

6. The process according to claim 5, wherein the base used for the arylation is sodium tert-butoxide.

7. The process according to any of the preceding claims, wherein the reaction is performed between 55 and 65 °C in toluene.

8. The process according to preceding claims, wherein the reaction is performed between 95 °C to the boiling temperature in dioxane.

9. The process according to any one of the preceding claims, wherein the compound of the formula (III) is prepared in a yield of at least 75% yield, preferably at least 85% yield, more preferably at least 90% yield.

10. The process according to any one preceding claims, wherein the compound of the formula (III), is converted by reaction with the aryl halide of the formula (V) or a salt thereof 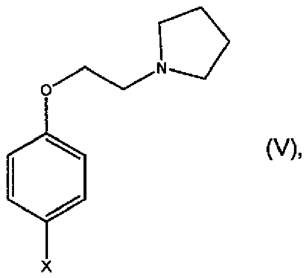 wherein X is selected from chlorine, bromine and iodine,
to yield a compound of the formula (VI) or a salt thereof, 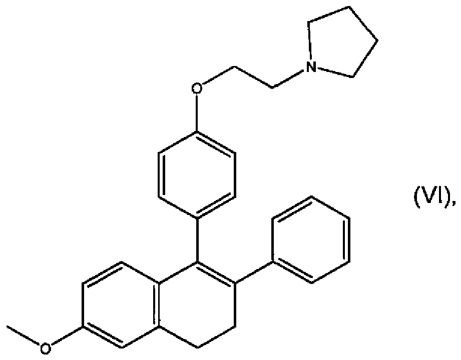 optionally converting the compound of the formula (VI) to a pharmaceutically acceptable salt.

11. Use of the compound of the formula (III) as intermediate for the preparation of nafoxidine or a salt thereof.

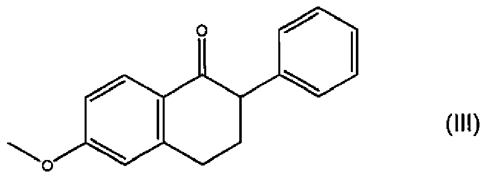

12. Use of the compound of the formula (III) as intermediate for the preparation of lasofoxifene or a salt thereof.

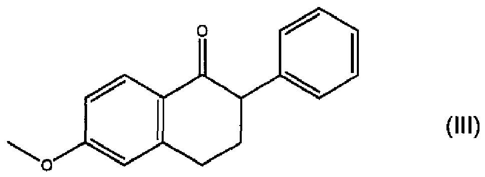

13. A process for the preparation of a compound of the formula (VII) or a salt thereof, comprising :
(i) converting a compound of the formula (I) to the arylated compound of the formula (III) or a salt thereof by a process according to any one of claim 1 to 10; 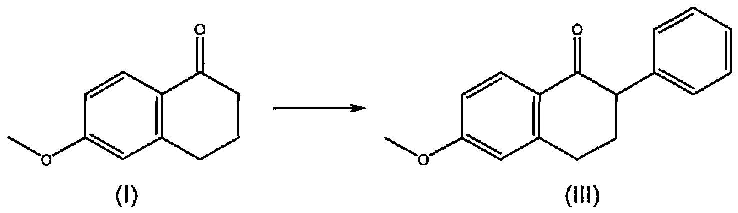
(ii) converting the compound of the formula (III) or a salt thereof by reaction with a compound of the formula (V) to prepare a compound of the formula (VI) or a salt thereof; 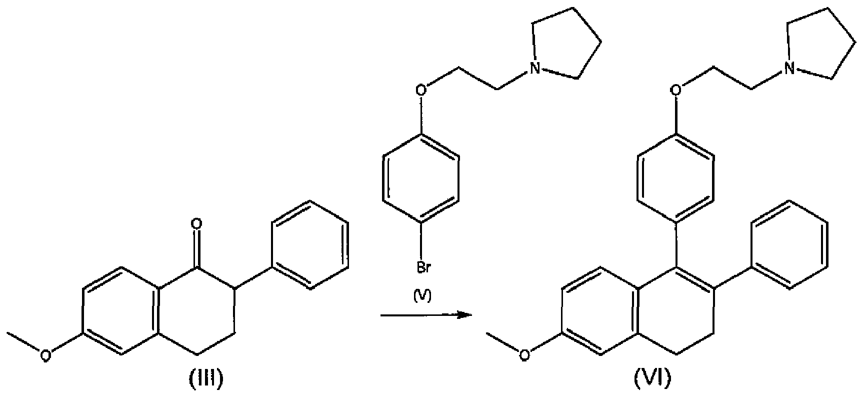
(iii) further converting the compound of the formula (VI) or a salt thereof to lasofoxifene or a salt thereof.

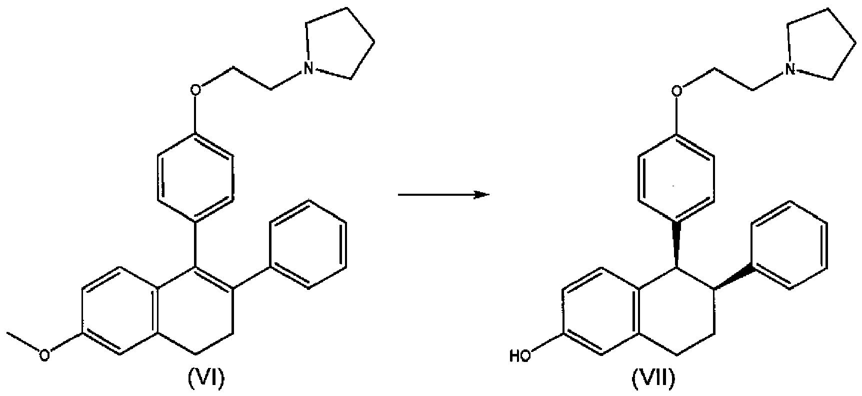

14. A process for the preparation of a pharmaceutical composition comprising nafoxidine or a salt thereof as active ingredient, comprising the steps of:
a) preparing nafoxidine or a salt thereof according to the process according to claim 10, and
b) admixing the prepared nafoxidine or a salt thereof with at least one pharmaceutically acceptable excipient.

15. A process for the preparation of a pharmaceutical composition comprising lasofoxifene or a salt thereof as active ingredient, comprising the steps of:
a) preparing lasofoxifene or a salt thereof according to the process according to claim 13, and
b) admixing the prepared lasofoxifene or a salt thereof with at least one pharmaceutically acceptable excipient.
